# EUROPEAN PATENT APPLICATION

(11) **EP 4 471 052 A1**
(43) Date of publication of application: **04.12.2024**
(21) Application number: 23745926.8
(22) Date of filing: 10.01.2023
(51) Int. Cl.: C07K 14/47, C07K 19/00, C12N 15/12, C12N 15/62, C12N 5/10, A61K 38/17, A61K 39/00, A61P 25/28

(54) **ESCHERICHIA COLI ENGINEERING STRAIN FOR FUSION EXPRESSION OF TAU-4R PROTEIN**

(30) Priority: 29.01.2022 CN 202210112904
(71) Applicant: Yuanben (Zhuhai Hengqin) Biotechnology Co., Ltd., Zhuhai, Guangdong 519000 (CN)
(72) Inventor: CAI, Jiong, Taizhou, Jiangsu 225300 (CN)
(74) Representative: Gille Hrabal Partnerschaftsgesellschaft mbB Patentanwälte
(86) International application number: PCT/CN2023/071534
(87) International publication number: WO 2023/143043

(57) **Abstract**

Disclosed in the present invention is fusion protein Tau-4R, which can be recombined into Escherichia coli to achieve the fusion expression. It is confirmed by means of experiments that the tau protein prepared by the strain may be used in the treatment research of Alzheimer's disease.

## Description

The present application claims priority to the prior application No. 2022101129048 entitled "ESCHERICHIA COLI ENGINEERING STRAIN FOR FUSION EXPRESSION OF TAU-4R PROTEIN" and filed to China National Intellectual Property Administration on Jan 29, 2022, which is incorporated herein by reference in its entirety.

### TECHNICAL FIELD

The present disclosure relates to the field of biomedicine, in particular to an *Escherichia coli* engineering strain for fusion expression of Tau-4R protein.

### BACKGROUND

Alzheimer's Disease (AD) is a neurodegenerative disease. The morbidity of the disease is extremely high in the elderly population, and the number of patients with AD gradually increases. In 2020, there were about 60 million AD patients in the world, and the number of AD patients will reach about 150 million by 2050.

Alzheimer's disease is the most typical disease of dementia and accounts for about 55% of all dementia cases. Alzheimer's disease shows two main pathological features, i.e., amyloid plaques and neurofibrillary tangles. Extracellular amyloid plaques are mainly composed of β amyloid peptides, and intracellular neurofibrillary tangles are mainly composed of Tau proteins. Also included are neurosynaptic dystrophy, astrocytosis, microglial activation, changes in mature neuronal markers, and the like. The clinical manifestations are cognitive impairment accompanied by memory decline. The drugs currently used for the treatment of AD mainly include two types of drugs, namely NMDA receptor antagonists (memantine) and acetylcholinesterase inhibitors (donepezil and galantamine), but the two types of drugs can only delay the symptoms of the disease but cannot reverse the disease process.

The amyloid-β cascade hypothesis of Alzheimer's disease believes that the accumulation of Aβ drives the development of AD. Although the hypothesis is strongly supported by the Aβ-related mutations and neurotoxicity of Aβ oligomers, Aβ plaques sometimes also appear in the brains of cognitively normal humans, leading to questioning of the Aβ cascade hypothesis. The hypothesis that Tau protein aggregation is the main driver of neurodegenerative diseases hasn't become widely accepted until recently. Hyperphosphorylated tau protein aggregates proliferate and spread between cells, and the tau pathology and the clinical symptoms of dementia have a better correlation than the Aβ pathology. Although the β amyloid antibody aducanumab (Biogen) approved in an accelerated manner by the U.S. FDA for the treatment of Alzheimer's disease reduces the amyloid β plaque load in patients, there are also side effects such as temporary swelling and headache of the brain area, confusion, dizziness, vision changes, nausea, angioedema, urticaria, amyloid-related imaging abnormalities, headache, falls, diarrhea, and being mentally disordered.

The Tau protein is a microtubule-associated protein involved in the development of the nervous system and is enriched around neuronal axons, and it binds to tubulin to form microtubules and can stabilize microtubules and promote axon transport. Under normal conditions, the Tau gene encodes 16 exons and is translated into Tau with 352-441 amino acids in length and 45-65 kDa in molecular weight. The Tau protein may be divided into an N-terminal projection functional domain (N-terminus), a proline-rich domain, a microtubule binding domain (MBD), and a C-terminal functional domain. The length of the N-terminus is uncertain, and the N-terminus can be inserted with extra fragments of exons 2 (E2) and 3 (E3). The proline-rich domain contains a large number of phosphorylation sites, while the C-terminus provides partial phosphorylation sites. The repetitive sequences (R1-R4) are located in MBD, have the strongest microtubule binding force, and promote self-aggregation of microtubules, and are also the core structure of the paired helical filament (PHF). Human Tau expresses 6 isoforms, namely Tau352, 381, 383, 410, 412, and 441, according to the mRNA editing mode, and the difference is whether 3 or 4 repetitive sequences exist at the C-terminus and whether 1 or 2 insertions exist at the N-terminus. Exon 10 (E10) encodes an R2 fragment. Tau proteins that retain E10 and have 4 repetitive sequences are collectively referred to as 4RTau, and Tau proteins that do not retain E10 and have only 3 repetitive sequences are collectively referred to as 3RTau. The expression levels of 3RTau and 4RTau in the adult brain are equivalent, and only 4RTau is expressed in adult mice.

Under pathological conditions, Tau undergoes conformational change, abnormal mRNA splicing, and the like, dissociates from microtubules, and forms pathological aggregation by itself through serial post-translational modification, thereby causing neurodegenerative diseases and finally forming Tau diseases.

### SUMMARY

To prevent or reverse the oligomerization of the tau protein, the inventors designed a fusion protein Tau-4R which can be recombined into *Escherichia coli* for fusion expression. It is confirmed by means of experiments that the tau protein prepared by the strain can be used in the treatment research of Alzheimer's disease.

Therefore, the following technical solution is adopted by the present disclosure:

The present disclosure provides a Tau-4R protein, which characterized in that an amino acid sequence of the Tau-4R protein is set forth in SEQ ID NO. 1.

The present disclosure further provides a fusion protein, which characterized in that the fusion protein comprises the Tau-4R protein according to the present disclosure. Preferably, the fusion protein comprises maltose-binding protein (MBP) and the Tau-4R protein according to the present disclosure.

According to the present disclosure, an amino acid sequence of the fusion protein is set forth in SEQ ID NO. 3.

The present disclosure further provides a derivative protein of the fusion protein, which characterized in that the derivative protein of the fusion protein is formed by adding or partially replacing with a linker on the basis of the fusion protein. Preferably, the linker is GGGGSGGGGS setting forth in SEQ ID NO. 5.

The linker of the present disclosure is a flexible short peptide sequence, which plays a role in increasing flexibility and thus allows reporter protein fragments to easily approach each other to achieve complementation.

According to the present disclosure, an amino acid sequence of the derivative protein of the fusion protein is set forth in SEQ ID NO. 4.

The present disclosure further provides a polynucleotide encoding the Tau-4R protein, the fusion protein, and/or the derivative protein of the fusion protein according to the present disclosure. Preferably, a sequence of the polynucleotide is set forth in SEQ ID NO. 2.

The present disclosure further provides a recombinant expression vector, which characterized in that the recombinant expression vector comprises the Tau-4R protein and/or the fusion protein thereof or the derivative protein of the fusion protein.

The present disclosure further provides a host cell, which characterized in that the host cell comprises the Tau-4R protein and/or the fusion protein thereof or the derivative protein of the fusion protein.

According to the present disclosure, the fusion protein is obtained by inducing a Tau-4R protein-expressing strain having a MBP tag.

Preferably, the fusion protein is induced by IPTG, and the strain is obtained by linking a vector having a MBP tag with a substance comprising a gene of the Tau-4 of the present disclosure and then transforming. Wherein, the substance comprising the gene of the Tau-4 of the present disclosure may be a PCR product or a plasmid. The vector having the MBP tag is selected from one or more of pMal-p5x, pMAL-c2G, pMAL-c2x, pMAL-c4x, and pET-28b-MBP-His.

The present disclosure provides a pharmaceutical composition, which comprises the Tau-4R protein and/or the fusion protein thereof or the derivative protein of the fusion protein according to the present disclosure.

The present disclosure further provides a method for preparing the Tau-4R protein described above. The present disclosure further provides a method for preparing the fusion protein or the derivative protein thereof described above, which comprises the following steps:
(1) obtaining a substance comprising a gene of the Tau-4R protein according to the present disclosure;
(2) linking a vector having a MBP tag with the substance comprising the Tau-4R protein obtained in step (1), transforming, and inducing expression to obtain the fusion protein.

According to the present disclosure, preferably, the substance comprising the gene of the Tau-4 of the present disclosure may be a PCR product or a plasmid. Preferably, in step (1), the substance comprising the gene of the Tau-4R protein according to the present disclosure is obtained by a molecular biological method or a chemical synthesis method. In step (2), the vector having the MBP tag is selected from one or more of pMal-p5x, pMAL-c2G, pMAL-c2x, pMAL-c4x, and pET-28b-MBP-His. The fusion protein is induced by IPTG.

The present disclosure further provides a method for purifying a fusion protein, which comprises the following steps: purifying the fusion protein using an amylose-resin affinity column. Preferably, after elution, an ion exchange column is used for elution.

According to the present disclosure, after the protein purified by the amylose-resin affinity column was eluted with 20 mM Tris-HCl (pH 7.4), a Q-Sepharose FF ion exchange column was used, followed by elution with 20 mM Tris-HCl and 200 mM NaCl (pH 7.4).

The present disclosure further provides use of the Tau-4R protein, the fusion protein, or the derivative protein of the fusion protein in manufacturing a medicament for treating a neurogenic disease.

According to the present disclosure, the neurogenic disease is a neurodegenerative disease; more preferably, the neurogenic disease is Alzheimer's disease.

The present disclosure further provides use of the Tau-4R protein, the fusion protein, or the derivative protein of the fusion protein in a medicament for prolonging the lifespan of a patient having a neurogenic disease. Preferably, the neurogenic disease is a neurodegenerative disease; more preferably, the neurogenic disease is Alzheimer's disease.

According to the use of the present disclosure, wherein an amino acid sequence of the Tau-4R protein is set forth in SEQ ID NO. 1.

According to the use of the present disclosure, wherein an amino acid sequence of the fusion protein is set forth in SEQ ID NO. 3.

According to the use of the present disclosure, wherein an amino acid sequence of the derivative protein of the fusion protein is set forth in SEQ ID NO. 4.

### Beneficial Effects of Present Disclosure:

1. The inventors design a fusion protein Tau-4R, which can be recombined into *Escherichia coli* to achieve fusion expression. After purification by the affinity column and the ion exchange column, the purity is greatly improved, and the production cost is greatly saved.
2. It is confirmed by means of experiments that the tau protein prepared by the strain can be used in the treatment research of Alzheimer's disease.

### BRIEF DESCRIPTION OF THE DRAWING

FIG. 1 shows the double enzyme digestion identification of the gene of Tau-4R (1. double enzyme digestion product; M: Marker, 10000 bp, 8000 bp, 6000 bp, 5000 bp, 4000 bp, 3500 bp, 3000 bp, 2500 bp, 2000 bp, 1500 bp, 1200 bp, 1000 bp, 900 bp, 800 bp, 700 bp, 600 bp, 500 bp, 400 bp, 300 bp, 200 bp, and 100 bp).
FIG. 2 shows the gene sequencing map of Tau-4R.
FIG. 3 shows the identification of the *Escherichia coli* strains for fusion expression of the Tau-4R protein (M: low molecular weight protein standard, 97.4 kD, 66.2 kD, 43 kD, 31 kD, 22 kD, and 14.4 kD;
   1: DH5α-pMal-p5x-Tau-4-1 strain before IPTG inducing;
   2: DH5α-pMal-p5x-Tau-4-1 strain after IPTG inducing;
   3: DH5α-pMal-p5x-Tau-4-2 strain before IPTG inducing;
   4: DH5α-pMal-p5x-Tau-4-2 strain after IPTG inducing;
   5: DH5α-pMal-p5x strain before IPTG inducing;
   6: DH5α-pMal-p5x strain after IPTG inducing.

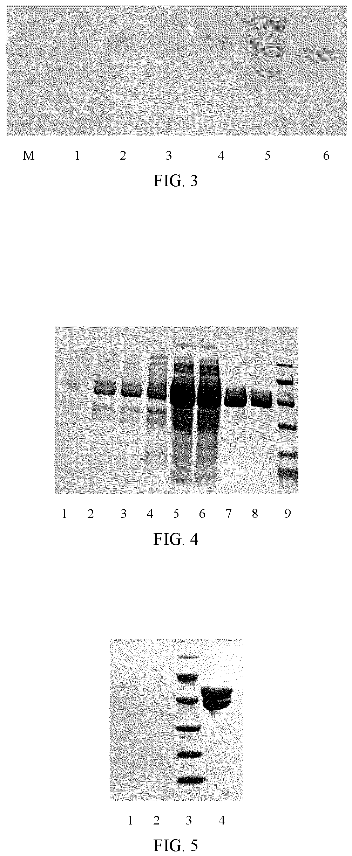

FIG. 4 shows the purification of the Tau-4R protein obtained by fusion expression by using bacterial cells (1: before inducing; 2: after inducing; 3: precipitation; 4: precipitation after ultrasonic treatment; 5: before loading of bacterial cell supernatant; 6: bacterial cell supernatant flow-through liquid; 7-8: elution of bacterial cell supernatant; 9: low molecular weight protein Marker, 97.4 KD, 66.2 KD, 43 KD, 31 KD, 22.0 KD, and 14.4 KD).
FIG. 5 shows the purification of the Tau-4R protein obtained by fusion expression by using medium (1: before loading of medium; 2: after loading; 3: Marker, 97.4 KD, 66.2 KD, 43 KD, 31 KD, 22.0 KD, and 14.4 KD; 4: eluent).

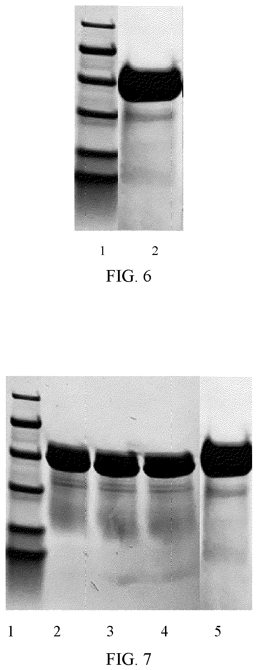

FIG. 6 shows the optimization of the process of the Tau-4R protein obtained by fusion expression (1: Marker, 97.4 KD, 66.2 KD, 43 KD, 31 KD, 22.0 KD, and 14.4 KD; 2: eluent).
FIG. 7 shows the storage of the Tau-4R protein obtained by fusion expression (1: Marker, 97.4 KD, 66.2 KD, 43 KD, 31 KD, 22.0 KD, and 14.4 KD; 2: storage at 4 °C after lyophilization; 3: storage at -20 °C; 4: storage at -80 °C; 5: before lyophilization).

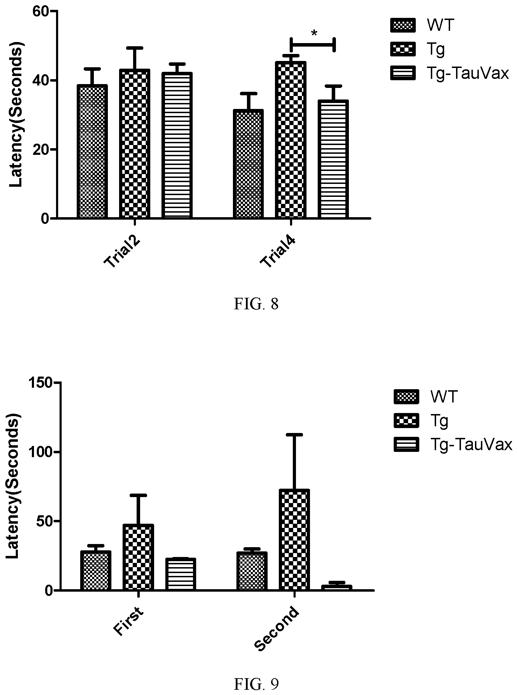

FIG. 8 shows that immunization of 3× transgenic AD mice with TauVax can shorten the latency (the time to jump onto the platform) in the fourth water maze exploration.
FIG. 9 shows the effect of immunization with TauVax on the latency (the time to jump onto the platform) of 3× transgenic AD mice in the step-down test.

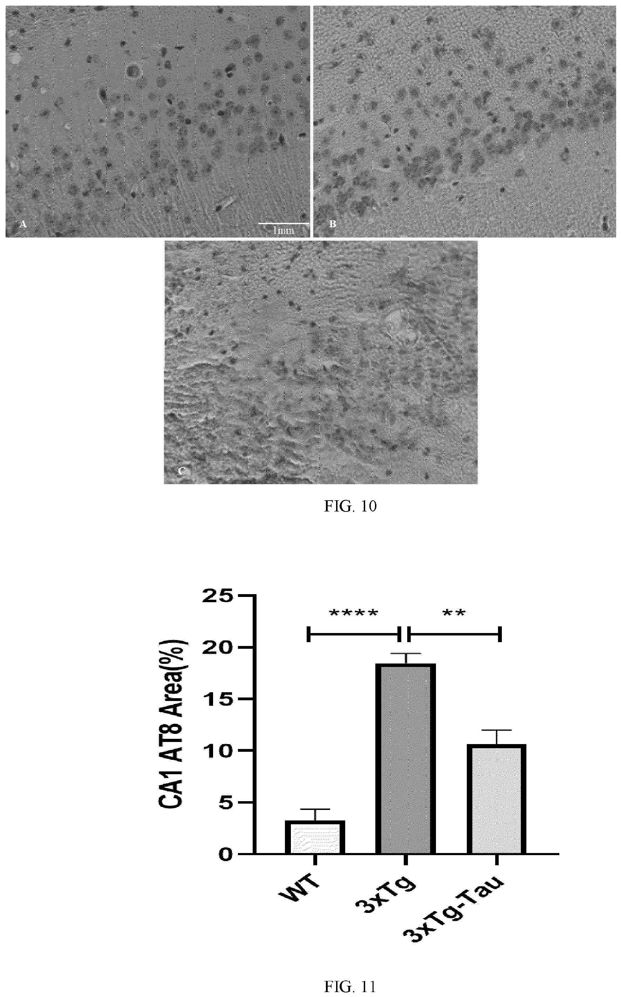

FIG. 10 shows that immunization of 3× transgenic AD mice with TauVax reduces amyloid plaque load in the hippocampus (A. wild-type mice; B. 3× transgenic mice treated with normal saline; C. 3× transgenic mice immunized with TauVax protein).
FIG. 11 shows that immunization with Tau-4R reduces phosphorylation of Tau protein in the cerebral hippocampus of 3 × transgenic mice.

### DETAILED DESCRIPTION

The technical solutions of the present disclosure will be further described in detail with reference to the following specific examples. It should be understood that the following examples are merely exemplary illustration and explanation of the present disclosure and should not be construed as limiting the protection scope of the present disclosure. All techniques implemented based on the content described above of the present disclosure are encompassed within the protection scope of the present disclosure.

Unless otherwise stated, the starting materials and reagents used in the following examples are all commercially available products or can be prepared by using known methods.

### Example 1. Design of Genes

The amino acid sequence SEQ ID NO. 1 of the recombinant protein Tau-4R is designed as follows: TENLKHQPGGGKGSKDNIKHVPGGGSGSLGNIHHKPGGGQGSLDNITHVPGGGN.

Through the optimization of the preferred codons of *Escherichia coli,* the following gene was designed, and the sequence SEQ ID NO. 2 is as follows:

CATATGACTGAAAATCTGAAACATCAACCTGGCGGCGGTAAAGGCTCTAAAGATAACA
TCAAGCACGTTCCTGGTGGCGGTTCTGGTTCCCTGGGTAACATCCATCACAAACCGGGT
GGCGGTCAAGGTAGCCTGGATAATATCACTCATGTGCCAGGTGGTGGCAATCCATGG.

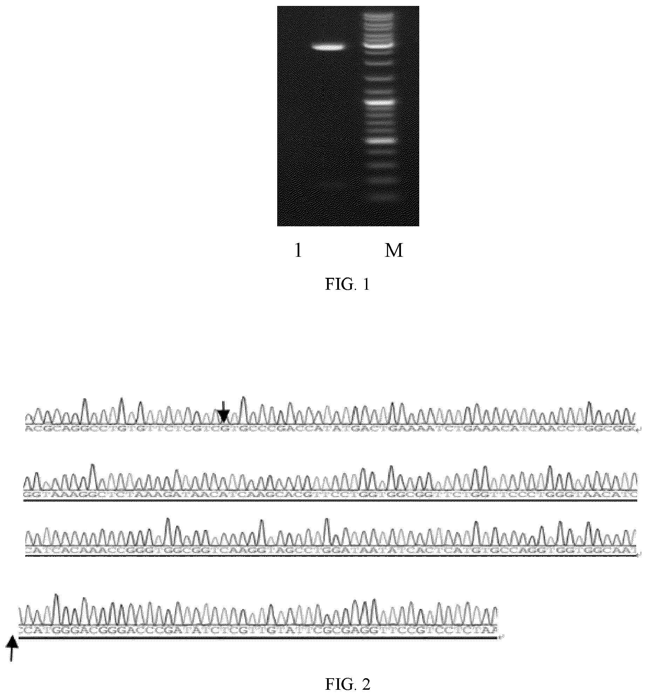

The gene has a NdeI digestion site at the 5' end and a NcoI digestion site at the 3' end. The gene was synthesized by multiplex PCR and transformed into the pUCm-T plasmid, followed by screening using an LB plate containing ampicillin; white colonies selected by IPTG/X-gal staining were subjected to PCR identification, followed by PCR identification and extraction of the plasmid for double enzyme digestion with NdeI and NcoI; it was determined that the size of the fragment was 100-200 bp, which was close to the theoretical value of 168 bp (FIG. 1).

The plasmid that was confirmed correct in digestion identification was sent for sequencing, and the results showed that the base sequence was completely correct, the length was correct, and the plasmid had a NdeI digestion site at the 5' end and a NcoI digestion site at the 3' end (FIG. 2).

### Example 2. Establishment of Escherichia coli Strain for Fusion Expression of Tau-4R Protein

Plasmids pUCm-T-Tau-4R and pMal-p5x were double enzyme digested with NdeI and NcoI, and small and large fragments of plasmids were collected, linked by a T4 ligase, and then transformed into DH5α competent bacteria, followed by screening using an LB plate containing ampicillin, PCR identification, and extraction of the plasmid for double enzyme digestion with NdeI and NcoI; it was determined that the size of the fragment was 100-200 bp, and thus the pMal-p5x-Tau-4R plasmid was obtained. Two clones were randomly selected, transferred to LB medium containing ampicillin separately, and incubated overnight at 37 °C and 220 rpm. The next day, the LB cultures were each transferred to 5 mL of LB medium containing ampicillin at a ratio of 1:1000 and incubated for 2.5 h at 37 °C and 220 rpm, followed by inducing overnight with 1 mM IPTG; a strain obtained by transforming competent DH5α with pMal-p5x empty plasmid was used as a negative control. The SDS-PAGE analysis was then performed.

The results showed that the strains DH5α-pMal-p5x-Tau-4-1 and DH5α-pMal-p5x-Tau-4-2 exhibited the expression of nascent proteins near 50 KD, and DH5α-pMal-p5x exhibited the expression of nascent proteins at 43 KD; *Escherichia coli* strains DH5α-pMal-p5x-Tau-4-1 and DH5α-pMal-p5x-Tau-4-2 that succeeded in the fusion expression of the Tau-4R protein were established.

### Example 3. Purification of MBP-Tau-4R Protein

The strain DH5α-pMal-p5x-Tau-4-1 was induced by 1 mM IPTG to obtain bacterial sludge containing the MBP-Tau-4R protein. The bacterial sludge was suspended in 20 mM Tris-HCl, 200 mM NaCl, and 1mM EDTA (pH 7.4), crushed by ultrasonication, centrifuged at 12000 rpm for 10 min (3 times), and filtered through a 0.45 µm filter membrane and a 0.22 µm filter membrane, followed by loading on an amylose-resin column and elution with 10 mM maltose, 20 mM Tris-HCl, 200 mM NaCl, and 1 mM EDTA (pH 7.4). The protein obtained was subjected to SDS-PAGE analysis.

The results showed that about 50 KD protein was obtained after purification, which was consistent with the theoretical molecular weight of the MBP-Tau-4R protein, but the purity of the target protein was only 34% (FIG. 4).

The bacterial culture supernatant was filtered through filter membranes (first 0.45 µm filter membrane and then 0.22 µm filter membrane), loaded on an amylose-resin column, and then eluted with 10 mM maltose, 20 mM Tris-HCl, 200 mM NaCl, and 1 mM EDTA (pH 7.4). The protein obtained was subjected to SDS-PAGE analysis. The results showed that about 50 KD protein was obtained after purification (FIG. 5). The purity of the target protein obtained in the culture medium was relatively high and was 60%.

The amino acid sequence SEQ ID NO.3 of MBP-Tau-4R is as follows:

KIEEGKLVIWINGDKGYNGLAEVGKKFEKDTGIKVTVEHPDKLEEKFPQVAATGDGPDIIF
WAHDRFGGYAQSGLLAEITPDKAFQDKLYPFTWDAVRYNGKLIAYPIAVEALSLIYNKDL
LPNPPKTWEEIPALDKELKAKGKSALMFNLQEPYFTWPLIAADGGYAFKYENGKYDIKDV
GVDNAGAKAGLTFLVDLIKNKHMNADTDYSIAEAAFNKGETAMTINGPWAWSNIDTSKV
NYGVTVLPTFKGQPSKPFVGVLSAGINAASPNKELAKEFLENYLLTDEGLEAVNKDKPLGA
VALKSYEEELVKDPRIAATMENAQKGEIMPNIPQMSAFWYAVRTAVINAASGRQTVDEAL
KDAQTNSSSNNNNNNNNNNLGIEGRISHMTENLKHQPGGGKGSKDNIKHVPGGGSGSLGN
IHHKPGGGQGSLDNITHVPGGGNPWAAAISSTDPNSLQVIK.

### Example 4. Derivatization of MBP-Tau-4R Protein

NNNNNNNNNN(SEQ ID NO. 6) was replaced with GGGGSGGGGS(SEQ ID NO. 5) to obtain a new derivative molecule, and the sequence SEQ ID NO. 4 is as follows:

KIEEGKLVIWINGDKGYNGLAEVGKKFEKDTGIKVTVEHPDKLEEKFPQVAATGDGPDIIF
WAHDRFGGYAQSGLLAEITPDKAFQDKLYPFTWDAVRYNGKLIAYPIAVEALSLIYNKDL
LPNPPKTWEEIPALDKELKAKGKSALMFNLQEPYFTWPLIAADGGYAFKYENGKYDIKDV
GVDNAGAKAGLTFLVDLIKNKHMNADTDYSIAEAAFNKGETAMTINGPWAWSNIDTSKV
NYGVTVLPTFKGQPSKPFVGVLSAGINAASPNKELAKEFLENYLLTDEGLEAVNKDKPLGA
VALKSYEEELVKDPRIAATMENAQKGEIMPNIPQMSAFWYAVRTAVINAASGRQTVDEAL
KDAQTNSSSGGGGSGGGGSLGIEGRISHMTENLKHQPGGGKGSKDNIKHVPGGGSGSLGNI
HHKPGGGQGSLDNITHVPGGGN.

### Example 5. Optimization of Process for Purification of MBP-Tau-4R Protein

The MBP-Tau-4R purified by an amylose-resin affinity column was dialyzed against 20 mM Tris-HCl (pH 7.4), and then a Q-Sepharose FF ion exchange column was used, followed by elution with 20 mM Tris-HCl and 200 mM NaCl (pH 7.4). The protein obtained had a higher purity (FIG. 6).

### Example 6. Screening of Storage Conditions for MBP-Tau-4R Protein

The MBP-Tau-4R protein was lyophilized and stored at 4 °C for 1 month, and this sample and samples that were not lyophilized and stored at -20 °C and -80 °C, respectively, for 1 month were subjected to SDS-PAGE analysis to determine the purity of the target protein.

**Table 1. Influence of different storage conditions on purity of Tau-4R protein obtained by fusion expression**

| | Storage at 4 °C after lyophilization | Storage at -20 °C | Storage at -80 °C | Before lyophilization |
|---|---|---|---|---|
| Main band grayscale | 21769 | 24245 | 24269 | 31867 |
| Impurity band grayscale | 597 | 295 | 94 | 396 |
| Main band purity (%) | 97.3 | 98.8 | 99.6 | 98.8 |

As shown in FIG. 7 and Table 1, no significant decrease in the purity of MBP-Tau-4R protein was observed.

### Example 7. Immunization of 3× Transgenic Mice with MBP-Tau-4R Protein and Water Maze Behavioural Test

The MBP-Tau-4R protein was adjusted to a concentration of 1 mg/mL and then mixed with an equal amount of 2% aluminum hydroxide adjuvant to obtain TauVax. 15-month-old 3× transgenic mice (APP/PS1/Tau) were immunized with TauVax by intramuscular injection of 100 µL of TauVax twice a week for 3 consecutive weeks. In this experiment, there were three groups, i.e., group 1 (six 15-month-old normal C57BL/6J mice, injection with normal saline), group 2 (six 15-month-old 3× transgenic mice, injection with normal saline), and group 3 (six 15-month-old 3× transgenic mice (APP/PS1/Tau), injection with TauVax). After the experiment was finished, the water maze behavioural test was carried out. The results showed that: after immunization with TauVax, the latency of the 3× transgenic AD mice in the second water maze exploration was not significantly affected, but the latency in the fourth water maze exploration could be shortened, with the latency reduced from 46.7±3.2 s to 34.8±7.9 s (p < 0.05) (FIG. 8). This suggests that the injection of TauVax can significantly improve the learning ability of 3× transgenic mice.

**Example 8.** Step-Down Test after Immunization of 3 × Transgenic Mice with MBP-Tau-4R Protein The specification of the four reaction chambers for the step-down test of mice was 25 cm × 25 cm × 30 cm, and a copper grid electrified with 36 V electricity for continuous electric stimulation was arranged at the bottom of each chamber. A rubber pad with a diameter of 12 cm and a height of 4.5 cm was placed in the middle of each chamber to serve as a safety zone for mice to avoid electric shock. Before the experiment, the mice were allowed to adapt to the environment for 3 min, and then the copper grid at the bottom was electrified with 36 V alternating current. The response time of the mouse jumping onto the rubber pad after receiving the electric stimulation was recorded as the learning score. After 24 h, the mouse was placed in the step-down apparatus again to adapt for 3 min and then placed on the rubber pad, and the latency of jumping onto the rubber pad after the mouse jumped off the platform for the first time was recorded and served as the memory score. The results showed that in the first test and the second memory reproducing test, the mice in the model group showed a significantly prolonged latency in jumping onto the platform compared with mice in the normal group. After treatment with TauVax, mice in the treatment group showed a shortened latency in jumping onto the platform (FIG. 9), suggesting that the learning and memory abilities of the 3× transgenic mice were reduced while the treatment with TauVax improved the learning and memory abilities of the 3× transgenic mice.

### Example 9. Immunohistochemical Staining after Immunization of 3× Transgenic Mice with MBP-Tau-4R Protein

Mice were subjected to perfusion after the behavioral test. On the first day, the heart perfusion was performed, and brain tissues were taken and immobilized with 4% paraformaldehyde in a refrigerator at 4 °C for 24 h. On the second day, the tissues were taken out from 4% paraformaldehyde and rinsed with tap water overnight. On the third day, the tissues were cut into pieces of suitable size, and the embedding cassettes were marked with a pencil and allowed to soak in alcohol jars (concentrations: 50%-75%-85%-95%-100%-TO transparentization agent; 1 h for each jar) and then in paraffin jars (I paraffin-II paraffin-III paraffin, I paraffin was oldest, III paraffin was newest, and the paraffin was placed in a 65 °C oven for 6-8 h in advance; 30 min for each jar), and finally the tissues were embedded in paraffin and stored in a refrigerator at 4 °C. On the fourth day, slicing (4 µm)-section drying (65 °C for 2 h) was performed, followed by transparentization with the TO transparentization agent (100%-95%-85%-75%-50%, dissolving in alcohol, 10 min for each jar) and antigen retrieval at 97.5 °C for 40 min (EDTA citrate retrieval solution), and then an endogenous peroxidase blocker (10% hydrogen peroxide) was added to cover the slides, which were incubated for 10 min at room temperature for blocking and then washed 3 times with PBS (5 min each time). PBS was removed, the slides were each placed on a glass slide, and normal goat serum working solution (1% normal goat serum) was added dropwise to each section, followed by incubation at room temperature for 10-15 min. Serum was removed, and a primary antibody 6E10 (amyloid1-16) or AT8 (S²⁰²T²⁰⁵) was added dropwise, followed by incubation overnight at 4 °C. On the fifth day, the section was washed 3 times with PBS, and a secondary antibody (biotin-labeled goat anti-mouse IgG) was added, followed by incubation at 37 °C for 60 min. The section was washed 3 times with PBS, and horseradish enzyme-labeled streptavidin (bound to the secondary antibody) was added dropwise, followed by incubation at room temperature for 10 min and washing 3 times with PBS (5 min/time). PBS was removed, and the fresh DAB solution was added dropwise (completed within 1-2 min, and immediately stopped when the section turned yellow). The section was soaked in tap water for 1-2 min and counterstained with hematoxylin for 1 min. After being soaked in 1% hydrochloric acid alcohol for 2-3 s (to remove non-specific staining), the slice was soaked in tap water and allowed to turn blue for about 15-30 min. Gradient dehydration in alcohol (50%-75%-85%-95%-100%), transparentization with the TO transparentization agent, and mounting with a mounting medium were performed successively. On the sixth day, the slides were dried and observed and photographed by using a microscope. The results showed that a large number of amyloid plaques were present in the cerebral hippocampus of the 3× transgenic mice, whereas the amyloid plaques were significantly reduced after treatment with TauVax (FIG. 10). Meanwhile, compared with the wild-type mice, the 3× transgenic mice were characterized by the presence of a large amount of phosphorylated Tau in the cerebral hippocampus and an increase of DAB staining area from 3.27±1.07% to 18.47±0.92% (p < 0.0001); after treatment with TauVax, phosphorylated Tau was significantly reduced, and DAB staining area decreased from 18.47±0.92% to 10.63±1.36% (p < 0.01) (FIG. 11).

Technical features in the above examples may be combined in any combinations. In order to make the description brief, all possible combinations of various technical features in the above examples are not described; however, it should be considered as being within the scope of this specification as long as there is no contradiction in the combinations of the technical features.

## Claims

1. A Tau-4R protein, which **characterized in that** an amino acid sequence of the Tau-4R protein is set forth in SEQ ID NO. 1.

2. A fusion protein or a derivative protein thereof, which **characterized in that** the fusion protein comprises the Tau-4R protein according to claim 1;
preferably, the fusion protein comprises MBP and the Tau-4R protein according to claim 1; preferably, an amino acid sequence of the fusion protein is set forth in SEQ ID NO. 3;
the derivative protein of the fusion protein is formed by adding or partially replacing with a linker on the basis of the fusion protein; preferably, the linker is GGGGSGGGGSGGGGS;
preferably, an amino acid sequence of the derivative protein of the fusion protein is set forth in SEQ ID NO. 4.

3. A polynucleotide which **characterized in** encoding the Tau-4R protein according to claim 1 or the fusion protein or the derivative protein thereof according to claim 2;
preferably, a sequence of the polynucleotide encoding the Tau-4R protein according to claim 1 is set forth in SEQ ID NO. 2.

4. A recombinant expression vector or a host cell expressing a gene of the Tau-4R protein according to claim 1 or the fusion protein or the derivative protein thereof according to claim 2;
preferably, a gene sequence of the Tau-4R protein is set forth in SEQ ID NO. 2.

5. A pharmaceutical composition, comprising the Tau-4R protein according to claim 1 or the fusion protein or the derivative protein thereof according to claim 2;
preferably, the pharmaceutical composition comprises a pharmaceutically acceptable carrier or adjuvant;
further preferably, the adjuvant is preferably an aluminum hydroxide adjuvant.

6. A method for preparing the Tau-4R protein according to claim 1, the fusion protein according to claim 2, and/or the derivative protein of the fusion protein according to claim 3.

7. The method according to claim 6, wherein the method for preparing the fusion protein comprises the following steps:
(1) obtaining a substance comprising a gene of the Tau-4R protein according to claim 1;
(2) linking a vector having a gene of a MBP tag with the substance comprising the gene of the Tau-4R protein obtained in step (1), transforming, and inducing expression to obtain the fusion protein;
preferably, the substance comprising the gene of the Tau-4R protein according to claim 1 can be a PCR product or a plasmid; preferably, in step (1), the substance of the gene of the Tau-4R protein is obtained by a molecular biological method or a chemical synthesis method;
preferably, in step (2), the vector having the MBP tag is selected from one or more of pMal-p5x, pMAL-c2G, pMAL-c2x, pMAL-c4x, and pET-28b-MBP-His;
preferably, the expression of the fusion protein is induced by IPTG.

8. A method for purifying the fusion protein or the derivative protein thereof according to claim 2, comprising the following steps:
purifying the fusion protein using an amylose-resin affinity column;
preferably, after elution, an ion exchange column is used for elution; more preferably, after the protein purified by the amylose-resin affinity column was eluted with 20 mM Tris-HCl (pH 7.4), a Q-Sepharose FF ion exchange column was used, followed by elution with 20 mM Tris-HCl and 200 mM NaCl (pH 7.4).

9. Use of the Tau-4R protein according to claim 1 or the fusion protein or the derivative protein thereof according to claim 2 in manufacturing a medicament for treating a neurogenic disease; preferably, the neurogenic disease is a neurodegenerative disease; more preferably, the neurogenic disease is Alzheimer's disease.

10. Use of the Tau-4R protein according to claim 1 or the fusion protein or the derivative protein thereof according to claim 2 in manufacturing a medicament for prolonging the lifespan of a patient having a neurogenic disease;
preferably, the neurogenic disease is a neurodegenerative disease; more preferably, the neurogenic disease is Alzheimer's disease.
